(19) Europäisches Patentamt
European Patent Office
Office européen des brevets

(11) Veröffentlichungsnummer : **0 137 251**
**B1**

(12) # EUROPÄISCHE PATENTSCHRIFT

(45) Veröffentlichungstag der Patentschrift : **28.09.88**

(51) Int. Cl.⁴ : **C 07 D498/04**, C 08 K 5/35 // (C07D498/04, 265:00, 265:00)

(21) Anmeldenummer : **84109977.3**

(22) Anmeldetag : **22.08.84**

(54) **Bismorpholine, Verfahren zu ihrer Herstellung und ihre Verwendung als Hydrolyseschutzmittel.**

(30) Priorität : **31.08.83 DE 3331436**

(43) Veröffentlichungstag der Anmeldung : **17.04.85 Patentblatt 85/16**

(45) Bekanntmachung des Hinweises auf die Patenterteilung : **28.09.88 Patentblatt 88/39**

(84) Benannte Vertragsstaaten : **BE DE FR GB IT**

(56) Entgegenhaltungen : **CHEMICAL ABSTRACTS, Band 87, Nr. 25, 19. Dezember 1977, Seite 732, Nr. 201553c, Columbus, Ohio, US**

(73) Patentinhaber : **BAYER AG**
**Konzernverwaltung RP Patentabteilung**
**D-5090 Leverkusen 1 Bayerwerk (DE)**

(72) Erfinder : **Haas, Peter, Dr.**
**Zwengenberger Strasse 43**
**D-5657 Haan 1 (DE)**
Erfinder : **Barnes, James Michael**
**Bechhausen 60**
**D-5653 Wermelskirchen (DE)**
Erfinder : **Goyert, Wilhelm, Dr.**
**Haberstrasse 48**
**D-5090 Leverkusen 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 137 251 B1

**Beschreibung**

Gegenstand der Erfindung sind neue 2,3,2',3'-Bismorpholinderivate der Formel

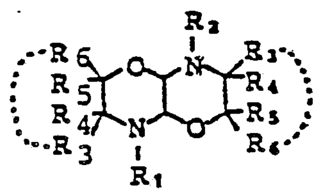

in welcher

$R_1$ und $R_2$ gleiche oder verschiedene $C_1$ bis $C_7$-Alkyl-, $C_5$-$C_6$-Cycloalkyl-, oder Phenylreste sind, wobei für $R_3$, $R_4$, $R_5$ und $R_6$ = Wasserstoff n-Butyl und Phenyl ausgenommen sind,

$R_3$, $R_4$, $R_5$, $R_6$ gleich oder verschieden Wasserstoff und/oder einen $C_1$-$C_7$-Alkyl-Substituenten, einen Phenylrest oder über Verknüpfung von $R_3$ und $R_6$ einen Cycloalkylen- oder Arylen-Substituenten (wobei für Arylen $R_4$, $R_5$ entfallen) bildend,

bedeuten.

Beansprucht wird auch ihr Herstellungsverfahren durch Umsetzung von Glyoxal mit N-monosubstituierten Aminoethanolen vom Typ

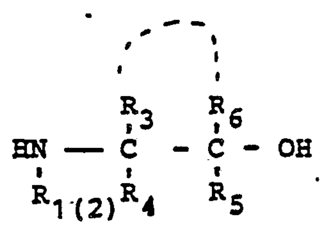

in wäßriger Lösung oder wasserhaltigen organischen Lösungsmitteln bei Temperaturen bis zum Siedepunkt des Wassers unter dem anliegenden Druck.

Erfindungsgegenstand ist ferner die Verwendung dieser 2,3,2',3'-Bismorpholinderivate in Mengen von 0,1 bis 10 Gew.-Teilen in ester- und/oder carbonatgruppenhaltigen Kunststoffen, insbesondere in Polyurethanen auf Basis von höhermolekularen Polyester- und/oder Polycarbonatpolyolen als Hydrolyseschutzmittel.

Bekanntlich sind Estergruppen (einschließlich Carbonatgruppen) gegen hydrolytische Spaltung unter Rückbildung der Ausgangskomponenten anfällig. Bei höhermolekularen Stoffen, die Estergruppen als Aufbaukomponenten enthalten, bewirkt eine solche Reaktion den Abbau der Polymerkette, was in der Regel einen Verlust der mechanischen Werte bewirkt. Ein weiterer Nachteil ist die autokatalytische Zerstörung durch fortwährend neu anfallende Carbonsäuregruppen, die immer drastischer wird.

Die Umsetzungsprodukte von Polyestern mit mindestens bifunktionellen Isocyanaten führt z. B. zu Polyurethanen, die als Hochleistungselastomere technisch mannigfaltige Anwendung finden. Um das hohe Werteniveau dieser Kunststoffe gegen einen Abbau durch feuchte Wärme, Wasser und Wasserdampf zu sichern, werden seit langem Hydrolyseschutzmittel verwendet, wie sie z. B. die Stoffgruppe der Carbodiimide darstellt und in der DE-PS 1 005 726 sowie den BE-PSen 610 969, 612 040 und 733 573 beschrieben sind. Aus der FR-PS 1 450 919 sind weiterhin Iminooxazolidine der Formel

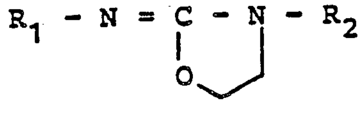

$R_1$ = Aryl

$R_2$ = Aryl, Alkyl

als Alterungsschutzmittel bei estergruppenhaltigen Verbindungen bekannt geworden. In beiden Fällen sind jedoch sowohl Wanderungstendenz, Auswirkungen auf den Druckverformungsrest als auch katalytische Wirkung (Veränderung der Gieß- und Topfzeiten von Polyurethanen) von Nachteil.

Die in der DE-PS 2 106 726 beschriebenen Hydroxyalkylharnstoff-Derivate zeigen zwar auch gute Wirkung, nachteilig sind jedoch hier die hohen Schmelzpunkte und die geringe Löslichkeit dieser Stoffklasse in Polyestern oder kurzkettigen Kettenverlängerern.

Aus CA, 87 (1977), Nr. 201553c, sind Verbindungen der allgemeinen Formel

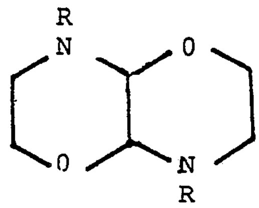
($R = C_4H_9$-, $C_6H_5$-)

bekannt. In der Veröffentlichung findet sich jedoch kein Hinweis auf deren Verwendung als Hydrolyseschutzmittel.

Völlig überraschend wurde nun eine Gruppe von Hydrolyseschutzmittel mit ausgeprägt positiv zu beurteilenden Verarbeitungseigenschaften aufgefunden, die mit den bisher dafür bekannten Stoffklassen in keinem Zusammenhang stehen, was Reaktionsverlauf, Struktur-und Stoffeigenschaften der Reaktionsprodukte betrifft.

Hierbei handelt es sich um als Hydrolyseschutzmittel (HSM) wirkende, bisher nicht beschriebene Reaktionsprodukte von Glyoxal und durch organische Gruppen substituierte Aminoethanolen, wobei Cycloacetale der Struktur von 2,3,2',3'-Bismorpholinen gemäß der Struktur

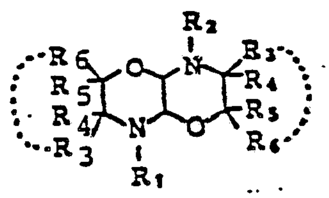

erhalten werden, in welchen

$R_1$ und $R_2$ gleiche oder verschiedene $C_1$ bis $C_7$-Alkyl-, $C_5$-$C_6$-Cycloalkyl-, oder Phenylreste sind, wobei für $R_3$, $R_4$, $R_5$ und $R_6$ = Wasserstoff n-Butyl und Phenyl ausgenommen sind,

$R_3$, $R_4$, $R_5$, $R_6$ gleich oder verschieden Wasserstoff und/oder einen $C_1$-$C_7$-Alkyl-Substituenten, einen Phenylrest oder über Verknüpfung von $R_3$ und $R_6$ einen Cycloalkylen- oder Arylen-Substituenten (wobei für Arylen $R_4$, $R_5$ entfallen) bildend, besonders bevorzugt Wasserstoff
bedeuten.

Besonders bevorzugt sind Verbindungen bei denen

$R_1$, $R_2$ eine $CH_3$-Gruppe, eine $C_2H_5$-Gruppe, eine Cyclohexylgruppe, eine Phenylgruppe wobei für $R_3$, $R_4$, $R_5$, $R_6$ = Wasserstoff die Phenylgruppe ausgenommen ist,

$R_3$, $R_4$, $R_5$, $R_6$ Wasserstoff oder die Reste

$R_3$ und $R_6$ über Verknüfung mit dem Aminoethanol einen Cyclohexanrest bilden,

$R_4$, $R_5$ Wasserstoff oder einen $C_1$ bis $C_7$-Alkylrest, vorzugsweise Wasserstoff
bedeutet.

Gegenstand der Erfindung ist auch ein Verfahren zur Herstellung der 2,3,2',3'-Bismorpholinderivate aus

a) Glyoxal und

b) N-monosubstituierten Aminoethanolen vom Typ

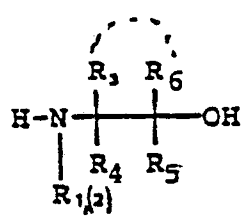

in welcher $R_{1,2}$ sowie $R_3$, $R_4$, $R_5$, $R_6$ die beschriebene Bedeutung haben, wobei im Falle von Arylenresten $R_4$, $R_5$ entfällt, durch Umsetzung der Komponenten (bevorzugtes Umsetzungsverhältnis etwa 1 : 2) in wäßriger Lösung oder in wasserhaltigen Lösungsmitteln bei Temperaturen bevorzugt bis zum Siedepunkt des Wassers unter dem anliegenden Druck, vorzugsweise 40 bis 100 °C.

Gegenstand der Erfindung ist ferner die Verwendung von 2,3,2',3'-Bismorpholinen als Hydrolyseschutzmittel für Estergruppen und/oder Carbonatgruppen enthaltende Kunststoffe, insbesondere Estergruppen und/oder Carbonatgruppen enthaltende Polyurethane aus Polyisocyanaten, Estergruppen und/oder Carbonatgruppen aufweisenden Polyolen, vorzugsweise höhermolekularen Polyolen, sowie gegebenenfalls weiteren Hilfs- und Zusatzstoffen wobei auch weitere, an sich übliche Hydrolyseschutzmittel mitverwendet werden können, indem man die 2,3,2',3'-Bismorpholinderivate der oben gekennzeichneten Struktur in Mengen von 0,1 bis 10, bevorzugt 0,3 bis 5, besonders bevorzugt 0,5 bis 2,5 Teilen, im Kunststoff verwendet.

Die Bismorpholine können aufgrund ihrer guten Löslichkeit den Ausgangsstoffen oder ihren Gemischen, bei Polyurethanen beispielsweise entweder dem höhermolekularen Polyolteil und/oder den üblich verwendeten Kettenverlängerungsmitteln oder ihren Gemischen zugesetzt werden. Sie können ferner auch den fertigen, Ester- und/oder Carbonatgruppen enthaltenden Kunststoffen, z. B. ihren Schmelzen, Lösungen oder Dispersionen, einverleibt werden.

Als erfindungsgemäße Vertreter dieser Stoffklasse der Bismorpholine seien angeführt :

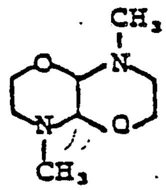

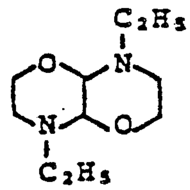

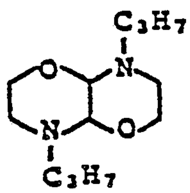

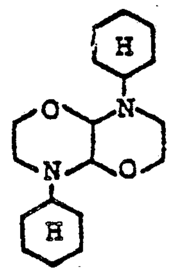

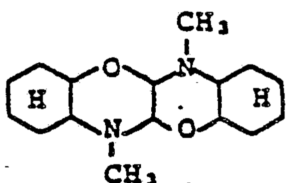

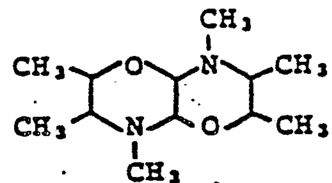

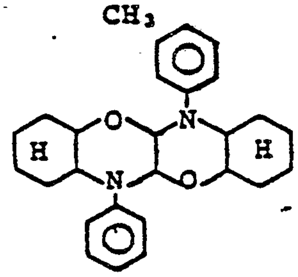

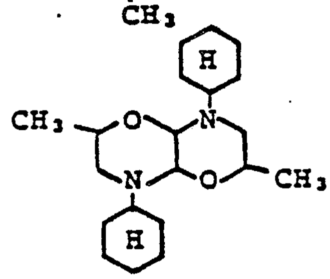

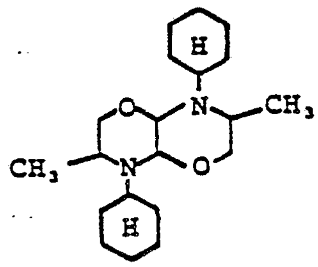

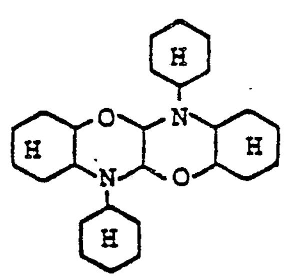

(Fortsetzung)

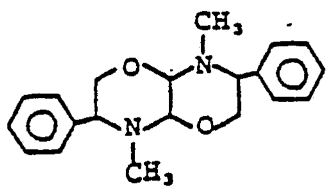

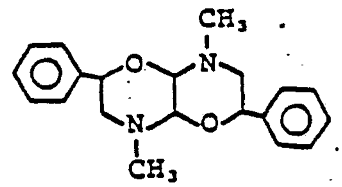

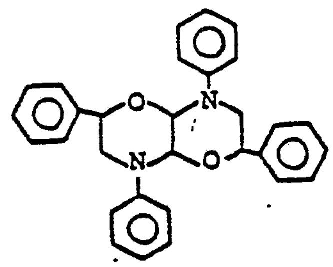

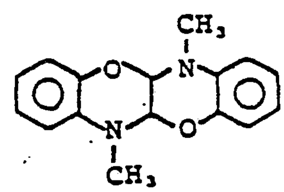

Als Ausgangsverbindungen für die Darstellung der Bismorpholine dient Glyoxal als bevorzugter Bisaldehyd, der im allgemeinen in Form seiner technischen, wäßrigen Lösung eingesetzt wird.

Als Beispiele für die N-monosubstituierten β-Aminoalkohole seien folgende Verbindungen angeführt : N-Methyl-ethanolamin, N-Ethyl-ethanolamin, N-Propyl-ethanolamin, N-Cyclohexyl-ethanolamin, 2-Hydroxyethyl-anilin, 2-(N-Methylamino)-buten-(3)-ol-(1), N-Methylamino-2-hydroxy-buten-(3), 2-(N-Phenylamino)-buten-(3)-ol-(1), N-Phenylamino-2-hydroxy-buten-(3), 2-(N-Methylamino)-2-phenyl-ethanol, 2-(N-Methylamino)-1-phenyl-ethanol, 2-(N-Phenylamino)-2-phenyl-ethanol, 2-(N-Phenylamino)-1-phenyl-ethanol, 2-(N-Phenylamino)-cyclohexanol-1, 2-(N-Cyclohexylamino)-cyclohexanol-(1), 1-(N-Phenyl)-2-hydroxypropylamin, 1-(N-Cyclohexyl)-2-hydroxypropylamin und 1-(N-Methyl)-2-hydroxypropylamin oder ihre Gemische.

Glyoxal und die β-Aminoalkohole werden bevorzugt in stöchiometrischen Mengen (1 : 2) umgesetzt ; bei Überschuß einer der Komponenten bilden sich trotzdem die erfindungsgemäßen Verbindungen entsprechend der Menge der Unterschußkomponente. Die Reaktion selbst wird bevorzugt in wäßriger Lösung durchgeführt, wobei nach kurzem Erwärmen, z. B. auf 40 bis 100 °C, die Reaktionsprodukte, besonders die der höher substituierten Vertreter, ausfallen, während z. B. die Methyl- oder Ethylderivate durch teilweises Einengen ihrer Lösung praktisch quantitativ mit guter Reinheit erhalten werden können. Die Reaktion kann auch in wäßrig-organischen Lösungsmitteln erfolgen, z. B. wäßrigen Alkoholen, Ethern oder Ketonen und ähnlichen Verbindungen.

Erfindungsgemäß können insbesondere Estergruppen und/oder Carbonatgruppen aufweisende Polyurethane wirksam stabilisiert werden. Unter Ester- und/oder Carbonatgruppen aufweisenden Polyurethanen sind beliebige, unter Mitverwendung von Estergruppen und/oder Carbonatgruppen aufweisenden Polyolen hergestellte Polyurethane, d. h. Polyurethanschaumstoffe, Polyurethanelastomere, Beschichtungsmittel auf Polyurethanbasis, Klebstoffe, Fugenvergußmassen und andere zu verstehen. Solche estergruppenhaltigen Polyurethane können auf der Basis von höhermolekularen Hydroxypolyestern, Hydroxypolylactonen, z. B. auf der Basis von ε-Caprolactonen, Hydroxypolycarbonaten, z. B. Hexandiolpolycarbonaten und ester- bzw. carbonatgruppenhaltigen Polyethern aufgebaut werden.

Außer zur bevorzugten Stabilisierung der Estergruppen und/oder Polycarbonatgruppen aufweisenden Polyurethane eignen sich die erfindungsgemäß zu verwendenden Hydrolyseschutzmittel auch zum Schutz beliebig anderer, Estergruppen und/oder Carbonatgruppen aufweisender Kunststoffe, wie z. B. Polyestern aus Polycarbonsäuren, vorzugsweise Dicarbonsäuren wie Phthalsäure, Isophthalsäure, Terephthalsäure, Adipinsäure oder Kohlensäure, und mehrwertigen Alkoholen, wie z. B. Ethylenglykol, Butandiol, Neopentyldiol, Hexandiol, Glycerin, 2,2-Bis-(4'-hydroxyphenyl)-propan, Trimethylolpropan, Pentaerythrit usw. Sie können auch zum Schutz von seitenständigen Estergruppen aufweisenden Polymeren bzw. Copolymeren auf Acrylsäure- bzw. Methacrylsäureesterbasis oder für Kunststoffe auf der Basis von Vinylestern, wie z. B. Polyvinylacetat usw. verwendet werden. Außerdem können Polycarbonate, z. B. auf der Basis von 2,2-Bis-(4'-hydroxyphenyl)-propan stabilisiert werden.

Derartige Kunststoffe können beispielsweise als Lacke, Folien, Überzüge, Fasern, Schaumstoffe, Elastomere, Gießharze oder Preßkörper vorliegen.

A) Herstellung der 2,3,2',3'-Bismorpholine (Verfahren und Eigenschaften).

Beispiel 1

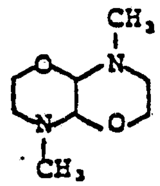

$C_8H_{16}N_2O_2$ (172)

In 375 g (5 mol) Methylethanolamin werden 482 g (2,5 mol) 30 % wäßrige Glyoxallösung getropft, wobei die Temperatur auf 75 bis 80 °C steigt. Man erhitzt 5 h auf 80 °C und engt danach die Lösung ein, wobei das Bismorpholin langsam auskristallisiert. Die Restfeuchtigkeit wird durch Abnutschen entfernt und das Produkt getrocknet. Ausbeute 735 g, entspr. 85 % d. Th. Schmp. 73-74 °C.

Das leicht gelbe Produkt kann durch Sublimation in eine völlig farblose Substanz vom Schmp. 75 °C überführt werden.

Analyse ber.: C 55,8 H 9,3 N 16,3;
gef.: C 55,2 H 9,0 N 16,2;

Im Massenspektrum wird das Molekülion $M^+$ bei 172 beobachtet. Zur Strukturaufklärung diente die Hochfeldresonanz, deren Ergebnisse tabellarisch aufgeführt sind.

$^1$H-Daten vom Beispiel 1, gem. bis 360 MHz in $CDCl_3$

| Chem. Versch. | (ppm) | Kopplungskonst. | | J (Hz) |
|---|---|---|---|---|
| $H_2a$ | 3,673 | 2a, | 2e | (-) 11,6 |
| $H_2e$ | 3,913 | 2e, | 3a | 3,6 |
| $H_3a$ | 2,893 | 2e, | 3e | 1,3 |
| $H_3e$ | 2,285 | 2a, | 3e | 3,0 |
| $H_5$ | 4,000 | 2a, | 3a | 11,5 |
| $NCH_3$ | 2,480 | 3a, | 3e | (-) 11,5 |

Die erhaltenen Daten entsprechen denen des Morpholinrings [1,2]. Das gilt vor allem für die geminalen und vicinalen Kopplungskonstanten.

Beispiel 2

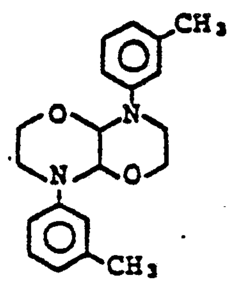

$C_{20}H_{20}N_2O_2$ (320)

In 48,5 g 30 %iger Glyoxallösung werden 75,5 g 3-Methyl-N-hydroxyethyl-anilin getropft, für 2 h auf 80 °C erwärmt, abgekühlt, abgesaugt; der Rückstand wird mit 120 ml Methanol warm aufgerührt, abgesaugt und getrocknet; Ausbeute 58 g farbl. Kristalle, Schm. 151-152 °C.

Analyse ber.: C 75,0 H 6,2 N 8,7;
gef.: C 74,3 H 7,8 N 8,5;

$^1$H-Daten in $CDCl_3$ bei 360 MHz für den Morpholinteil

[1]) W. B. Smith u. B. A. Shoulders, J. Phys. Chem 69, 579 (1965).

[2]) J. Devilliers, H. D. Giao und J. Novech, Compt. Rend. 277C, 1067 (1973).

| Chem. Versch. | (ppm) | Kopplungskonst. | | J (Hz) |
|---|---|---|---|---|
| $H_2a$ | 3,857 | 2a, | 2e | - 11,6 |
| $H_2e$ | 4,073 | 2e, | 3a | 3,6 |
| $H_3a$ | 3,500 | 2e, | 3e | 1,4 |
| $H_3e$ | 3,077 | 2a, | 3e | 2,9 |
| $H_5$ | 5,062 | 2a, | 3a | 11,5 |
| | | 3a, | 3e | - 11,9 |

Die Struktur als Morpholinderivat wird demnach auch wie unter Beispiel 1 belegt.

Beispiel 3

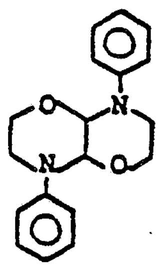

$C_{18}H_{20}N_2O_2$ (296)

In 96,8 g 30 %iger Glyoxallösung werden 137 g Hydroxyethylanilin getropft, für 2 h auf 80 °C erwärmt, abgekühlt, abgesaugt, in 250 ml Methanol erwärmt, abgesaugt und getrocknet ; Ausbeute 89 g farblose Kristalle, Schmp. 163 °C ;

Analyse ber. : C 73,0 H 6,7 N 9,45 ;
gef. : C 71,4 H 6,5 N 8,9 ;

Beispiel 4

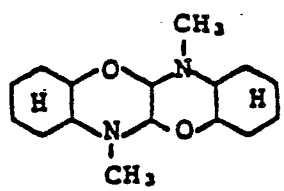

$C_{16}H_{20}N_2O_2$ (280)

In 48,5 g 30 %iger wäßriger Glyoxallösung werden 65 g 2-Aminomethylcyclohexanol getropft, 3 h auf 80 °C erwärmt, anschließend eingeengt, auf Ton getrocknet und aus 70 ml Methanol umkristallisiert ; Ausbeute 24 g farblose Kristalle, Schmp. 111-113 °C

Analyse ber. : C 68,5 H 10,0 N 10,0 ;
gef. : C 68,8 H 9,0 N 9,9 ;

Beispiel 5

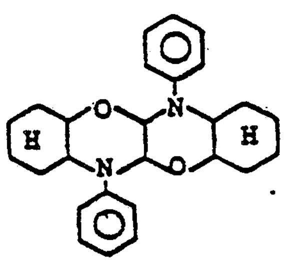

$C_{26}H_{32}N_2O_2$ (404)

19,4 g 30 %ige Glyoxallösung und 38,2 g Aminophenylcyclohexanol werden für 2,5 h auf 100 °C erwärmt, von Wasser abdekantiert, in wenig Methanol angerührt, abgesaugt und getrocknet ; Ausbeute 15 g farblose Kristalle, Schmp. 198-199 °C

Analyse ber. : C 77,2 H 7,9 N 6,95 ;
gef. : C 76,0 H 7,5 N 6,5 ;

Beispiel 6

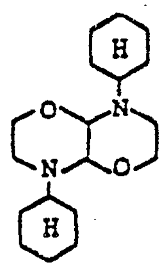

$C_{18}H_{32}N_2O_2$ (308)

In 38,8 g 30 %iger Glyoxallösung werden bei 25 °C 57,2 g Hydroxyethylcyclohexylamin getropft und für 4 h auf 70 °C erwärmt. Nach dem Absaugen wird aus Methanol umkristallisiert, Ausbeute 44 g, 72 % der Theorie, farblose Nadeln, Schmp. 95-97 °C.

Analyse ber : C 70,0 H 10,4 N 9,1 ;
gef : C 69,9 H 10,0 N 9,1 ;

B) Verwendung als Hydrolyseschutzmittel

Beispiel 7

7.1 Standard-Polyurethan ohne Hydrolysenschutzmittel (Vergleich)

100 Gew.-Teile eines aus Adipinsäure und Ethylenglykol bestehenden Polyesters mit einem mittleren Molekulargewicht von 2.000 und einer OH-Zahl von 56 werden bei 130 °C unter einem Vakuum von ca. 60 mbar (40 mm Hg) entwässert und anschließend bei ca. 130 °C gehalten.

Dann gibt man 25 Gew.-Teile 1,5-Diisocyanatonaphthalin zu. Nach ca. 2 Minuten wird ein Vakuum von ca. 60 mbar (40 mm Hg) angelegt. Nach Beendigung der exothermen Reaktion wird zu dem Prepolymer unter starkem Rühren 5 Gew.-Teile Butandiol-1,4 zugegeben. Es wird ca. 30 Sekunden gerührt und in eine auf 110 °C vorgeheizte Form gegossen. Die Mischung ist ca. 60 Sekunden gießfähig und erstarrt nach ca. 9 Minuten.

Das Elastomer wird noch 24 Stunden bei 110 °C getempert. Danach hat es die in Tabelle 1) angegebenen Eigenschaften.

Nach einer Lagerung in Wasser bei 80 °C (Lagerkörper — Normstab S1 gemäß DIN 53504) wird die Zugfestigkeit, Spannung bei 100 % und die Reißdehnung nach 0, 7, 9 und 11 Tagen erneut gemessen (siehe Tabelle 2).

Beispiel 8

Verwendung als Hydrolyseschutzmittel in Polyurethanelastomeren

Wie in Beispiel 7 wird das NCO-Prepolymer hergestellt. Nach Beendigung der exothermen Reaktion wird zu dem NCO-Prepolymer unter starkem Rühren eine Abmischung von 0,6 Gew.-Teilen, 4,4'-Dimethyl-2,3, 2', 3'-bismorpholin nach Beispiel 1 in 5 Gew.-Teile Butandiol 1,4 wie gegeben und wie in Beispiel 7 verarbeitet.

Nach einer Lagerung in Wasser bei 80 °C (Lagerkörper — Normstab S1 gemäß DIN 53 504) wird die Zugfestigkeit, Spannung bei 100 % und die Reißdehnung erneut gemessen (siehe Tabelle 2).

Die stabilisierende Wirkung der 2,3,2',3'-Bismorpholine zeigt sich besonders deutlich nach der 11-tägigen Einwirkung von Wasser bei 80 °C bei den Festigkeits- und Dehnungswerten.

Beispiel 9

(Vergleich mit einem bekannten Hydrolyseschutzmittel)

Verwendet man statt der erfindungsgemäßen Verbindung nach Beispiel 8 0,85 Teile 2,2',6,6'-Tetraisopropyldiphenylcarbodiimid, werden folgende Werte im Hydrolysetest erhalten (siehe Tabelle 2).

Die Vergleichswerte zeigen, daß die erfindungsgemäße Stoffklasse in ihrer Wirkung einem üblichen, technisch verbreitet eingesetzten Stabilisator überlegen ist.

Beispiel 10

Das NCO-Prepolymer wird wie in Beispiel 7 hergestellt. Nach Beendigung der exothermen Reaktion wird zu dem Prepolymer unter starkem Rühren eine Abmischung von 0,8 Gew.-Teilen nach Beispiel 6 und 5,0 Gew.-Teilen Butandiol-1,4 zugegeben. Es wird ca. 30 Sekunden gerührt und in eine auf 110 °C vorgeheizte Form gegossen. Die Mischung ist ca. 50 Sekunden gießfähig und erstarrt nach ca. 9 Minuten.

Das Elastomer wird noch 24 Stunden bei 110 °C getempert. Danach hat es folgende Eigenschaften (siehe Tabelle 1).

Nach einer Lagerung in Wasser bei 80 °C (Lagerkörper — Normstab S1 gemäß DIN 53504) wird die Zugfestigkeit, Spannung bei 100 % und die Reißdehnung erneut gemessen (siehe Tabelle 2).

Tabelle 1

Eigenschaften der Polyester-Polyurethane (Vor Hydrolyse)

| | Maßeinheit | Prüfnorm nach DIN | Beispiel Nr. | | | | | | |
|---|---|---|---|---|---|---|---|---|---|
| | | | 7 (Vergleich) | 8 | 9 (Vergleich) | 10 | 11 | 12 | 13 |
| Shore-Härte A/D | - | 53505 | 91/37 | 90/35 | 90/36 | - | 85 | 90 | 85 |
| Spannung bei 100 % | MPa | 53504 | 8,65 | 8,26 | 8,44 | - | - | - | - |
| Zugfestigkeit | MPa | 53504 | 47,0 | 44,5 | 46,7 | - | 39 | 49,6 | 38 |
| Reißdehnung | % | 53504 | 719 | 700 | 716 | - | 570 | 550 | 580 |
| Weiterreißfestigkeit | kN/m | 53513 | 73 | 78 | 77 | - | - | - | - |
| Stoßelastizität | % | 53512 | 54 | 53 | 54 | - | 46 | 49 | 47 |
| Weiterreißwiderstand | kN/m | 53515 | - | - | - | - | 78 | - | 77 |

Tabelle 2

Eigenschaften nach Hydrolyse in Wasser

| | | Maßeinheit | Prüfnorm nach DIN | Lagerkörper, Normstab S1 nach DIN 53504 Lagerung in Wasser bei 80°C in Tagen (d) nach 0d | 7d | 9d | 11d |
|---|---|---|---|---|---|---|---|
| Beispiel 7 | Spannung bei 100% | MPa | 53504 | 8,65 | 6,82 | 6,07 | 5,9 |
| (Vergleich | Zugfestigkeit | MPa | 53504 | 47,0 | 27,8 | 11,1 | 8,8 |
| ohne Hydr. Schutzmittel | Reißdehnung | % | 53504 | 719 | 690 | 365 | 266 |
| Beispiel 8 | Spannung bei 100% | MPa | 53504 | 8,26 | 6,94 | 6,26 | 6,42 |
| (erfindungs- | Zugfestigkeit | MPa | 53504 | 44,5 | 34,7 | 25,6 | 22,5 |
| gemäß) | Reißdehnung | % | 53504 | 700 | 778 | 691 | 634 |
| Beispiel 9 | Spannung bei 100% | MPa | 53504 | 8,44 | 6,78 | 6,44 | 6,40 |
| Vergl. mit | Zugfestigkeit | MPa | 53504 | 46,7 | 30,4 | 23,9 | 20,7 |
| Stand der Technik | Reißdehnung | % | 53504 | 716 | 760 | 701 | 659 |
| Beispiel 10 | Spannung bei 100% | MPa | 53504 | | | | |
| (erfindungs- | Zugfestigkeit | MPa | 53504 | gute Hydrolysen-Stabilität | | | |
| gemäß) | Reißdehnung | % | 53504 | | | | |

Tabelle 2a

Eigenschaften nach Hydrolyse in Wasser

| | | Maßeinheit | Prüfnorm nach DIN | Hydrolysentest nach x Tagen bei 100°C in nach 0d | nach 6d |
|---|---|---|---|---|---|
| Beispiel 11 (erfindungsgemäß) | Zugfestigkeit | MPa | 53504 | 39,0 | 32,0 |
| | Reißdehnung | % | 53504 | 570 | 700 |
| Beispiel 12 (erfindungsgemäß) | Zugfestigkeit | MPa | 53504 | 49,6 | 20,5 |
| | Reißdehnung | % | 53504 | 550 | 688 |
| Beispiel 13 Vergleich ohne Zusatz | Zugfestigkeit | MPa | 53504 | 38,0 | 4,0 |
| | Reißdehnung | % | 53504 | 580 | 15 |

Beispiel 11

100 Gew.-Teile eines Butandiol-1,4/Adipinsäurepolyesters (OH-Zahl 49,1) werden mit 1 Gew.-Teil Hydrolysenschutzmittel gemäß Beispiel 1, 0,8 Gew.-Teile Stearylamid und 10 Gew.-Teile Butandiol-1,4 bei 120 °C verrührt. 39,6 Gew.-Teile 4,4'-Diisocyanatodiphenylmethan (Verhältnis von NCO/OH-Gruppen = 1,03) werden auf 60 °C erhitzt und unter starkem Rühren zu der obigen 120 °C warmen Lösung zugegeben. Das reagierende Gemisch wird anschließend auf ein mit Treennwachs versehenes Blech gegossen. Es verfestigt sich sehr rasch und kann bereits nach etwa 6 Minuten entformt werden. Das Polymer wird nun in Schneidemühlen zerkleinert und nach 2-4 Tagen zu Förmkörpern verspritzt, die 17 h bei 80 °C getempert werden. Diese Formkörper weisen die in Tabelle 1 aufgeführten physikalischen Daten auf.

Es fällt insbesondere die gute Hydrolysenstabilität dieses Produktes auf (Tabelle 2a). Nach 6 Tagen bei 100 °C ist dieses Elastomer technisch noch weitgehend intakt, während das Vergleichsbeispiel ohne 4,4'-Dimethyl-2,3,2',3'-bis-morpholin fast vollständig zerstört ist.

Beispiel 12

100 Gew.-Teile eines Butandiol-1,4/Adipinsäurepolyesters (OH-Zahl 49,1) werden mit 0,5 Gew.-Teilen des Hydrolysenschutzmittels gemäß Beispiel 6, 0,8 Gew.-Teilen Stearylamid und 10 Gew.-Teilen Butandiol-1,4 bei 120 °C verrührt. 39,6 Gew.-Teile 4,4'-Diisocyanatodiphenylmethan (Verhältnis von NCO/OH-Gruppen = 1,03) werden auf 60 °C erhitzt und unter starkem Rühren zu der obigen 120 °C warmen Lösung zugegeben. Das reagierende Gemisch wird anschließend auf ein mit Trennwachs versehenes Blech gegossen. Es verfestigt sich sehr rasch und kann bereits nach etwa 6 Minuten entformt werden. Das Polymer wird nun in Schneidemühlen zerkleinert und nach 2-4 Tagen zu Formkörpern verspritzt, die 17 h bei 80 °C getempert werden. Diese Formkörper weisen die in Tabelle 1 aufgeführten physikalischen Daten auf.

Es fällt insbesondere die gute Hydrolysenstabilität dieses Produktes auf (Tabelle 2a). Nach 6 Tagen bei 100 °C ist dieses Elastomer technisch noch weitgehend intakt, während das Vergleichsbeispiel ohne 4,4'-Bis-cyclohexyl-2,3,2',3'-bismorpholin fast vollständig zerstört ist.

Beispiel 13

Es wird wie in Beispiel 11 und 12 verfahren. Es fehlt jedoch der erfindungsgemäße Stabilisatorzusatz. Die physikalischen Prüfwerte dieses verspritzten Thermoplasten sind in Tabellen 1 und 2a aufgeführt.

**Patentansprüche**

1. 2,3,2',3'Bismorpholinderivate der Formel

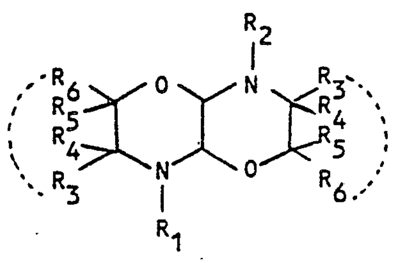

in welcher

$R_1$ und $R_2$ gleiche oder verschiedene $C_1$ bis $C_7$-Alkyl-, $C_5$-$C_6$-Cycloalkyl-, oder Phenylreste sind, wobei für $R_3$, $R_4$, $R_5$ und $R_6$ = Wasserstoff n-Butyl und Phenyl ausgenommen sind,

$R_3$, $R_4$, $R_5$, $R_6$ gleich oder verschieden Wasserstoff und/oder einen $C_1$-$C_7$-Alkyl-Substituenten, einen Phenylrest oder über Verknüpfung von $R_3$ und $R_6$ einen Cycloalkylen- oder Arylen-Substituenten (wobei für Arylen $R_4$, $R_5$ entfallen) bildend,

bedeuten.

2. Verbindungen nach Anspruch 1, dadurch gekennzeichnet, daß

$R_1$, $R_2$ eine $CH_3$-Gruppe, eine $C_2H_5$-Gruppe, eine Cyclohexylgruppe, eine Phenylgruppe wobei für $R_3$, $R_4$, $R_5$, $R_6$ = Wasserstoff die Phenylgruppe ausgenommen ist,

$R_3$, $R_4$, $R_5$, $R_6$ Wasserstoff oder die Reste

$R_3$ und $R_6$ über Verknüpfung mit dem Aminoethanol einen Cyclohexanrest bilden,

$R_4$, $R_5$ Wasserstoff oder einen $C_1$ bis $C_7$-Alkylrest, vorzugsweise Wasserstoff

bedeutet.

3. Verbindungen nach Ansprüchen 1 und 2, dadurch gekennzeichnet, daß
$R_1$ und $R_2$ = Methyl oder Cyclohexyl und
$R_3$, $R_4$, $R_5$, $R_6$ = H, oder
$R_3$, $R_6$ = $-(CH_2)_4-$
$R_4$, $R_5$ = H.
bedeutet.

4. Verfahren zur Herstellung der Verbindungen nach einem der Ansprüche 1 bis 3, dadurch gekennzeichnet, daß Glyoxal mit N-monosubstituierten Aminoalkoholen der Formel

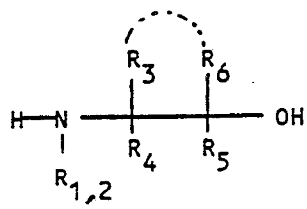

in welcher
$R_1$-$R_6$ die Bedeutung von Anspruch 1, 2 oder 3 haben, in wäßriger Lösung oder wasserhaltigen Lösungsmitteln, bevorzugt bei 40 bis 100 °C und einem bevorzugten Molverhältnis von 1 : 2 umsetzt.

5. Verwendung der erfindungsgemäßen Verbindungen nach den Ansprüchen 1 bis 3 in Mengen von 0,1 bis 10 Gew.-% im Kunststoff als Hydrolyseschutzmittel in Estergruppen und/oder Carbonatgruppen enthaltenden Kunststoffen.

6. Verwendung nach Anspruch 5 in Estergruppen und/oder Carbonatgruppen enthaltenden Polyurethanen aus Polyisocyanaten, Estergruppen und/oder Carbonatgruppen aufweisenden Polyolen sowie gegebenenfalls weiteren Hilfs- und Zusatzmitteln, dadurch gekennzeichnet, daß als Hydrolyseschutzmittel 2,3,2', 3'-Bismorpholine nach Ansprüchen 1 bis 3 in Mengen von 0,1 bis 10, bezogen auf das Polyurethan, verwendet werden.

7. Verwendung der erfindungsgemäßen Bismorpholine nach Ansprüchen 1 bis 3 als Hydrolyseschutzmittel unter Mitverwendung üblicher Hydrolyseschutzmittel.

**Claims**

1. 2,3,2',3'-Bismorpholine derivatives corresponding to the following formula

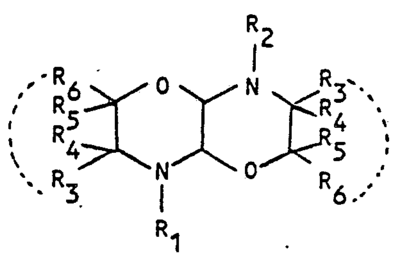

wherein
$R_1$ and $R_2$ denote identical or different $C_1$ to $C_7$-alkyl, $C_5$-$C_6$-cycloalkyl or phenyl groups but n-butyl and phenyl are excluded when $R_3$, $R_4$, $R_5$ and $R_6$ = hydrogen,
$R_3$, $R_4$, $R_5$ and $R_6$ may be identical or different and denote hydrogen and/or a $C_1$-$C_7$-alkyl substituent or a phenyl group or $R_3$ and $R_6$ are joined together to form a cycloalkylene or arylene or arylene substituent ($R_4$ and $R_5$ being absent in the case of arylene).

2. Compounds according to claim 1, characterised in that
$R^1$ and $R^2$ denote a $CH_3$ group, a $C_2H_5$ group, a cyclohexyl group or a phenyl group, the phenyl group being excluded when $R_3$, $R_4$, $R_5$, $R_6$ = hydrogen,
$R_3$, $R_4$, $R_5$, $R_6$ denote hydrogen or the groups
$R_3$ and $R_6$ are joined to the aminoethanol to form a cyclohexane group, and
$R_4$ and $R_5$ denote hydrogen or a $C_1$ to $C_7$-alkyl group, preferably hydrogen.

3. Compounds according to claims 1 and 2, characterised in that
$R_1$ and $R_2$ = methyl or cyclohexyl and
$R_3$, $R_4$, $R_5$, $R_6$ = H or
$R_3$, $R_6$, = $-(CH_2)_4-$ and
$R_4$, $R_5$ = H.

4. Process for the preparation of the compounds according to one of the claims 1 to 3, characterised in that glyoxal is reacted with N-monosubstituted aminoalcohols corresponding to the following formula

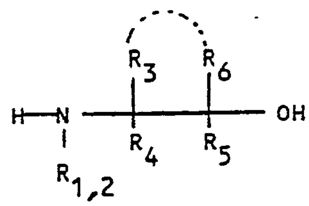

wherein

$R_1$-$R_6$ have the meanings indicated in claim 1, 2 or 3, in an aqueous solution or solvents containing water, preferably at 40 to 100 °C and in a preferred molar ratio of 1 : 2.

5. Use of the compounds according to the invention according to claims 1 to 3 in quantities of from 0.1 to 10 % by weight in the synthetic resin to serve as protective agent against hydrolysis in synthetic resins containing ester groups and/or carbonate groups.

6. Use according to claim 5 in polyurethanes which contain ester groups and/or carbonate groups and have been obtained from polyisocyanates, polyols containing ester groups and/or carbonate groups and optionally further auxiliary agents and additives, characterised in that the substances used for protecting against hydrolysis are 2,3,2',3'-bismorpholines according to claims 1 to 3 in quantities of from 0.1 to 10, based on the polyurethane.

7. Use of the bismorpholines according to the invention according to claims 1 to 3 as protective agents against hydrolysis together with conventional protective agents against hydrolysis.

**Revendications**

1. Dérivés de la 2,3,2',3'-bis-morpholine de formule

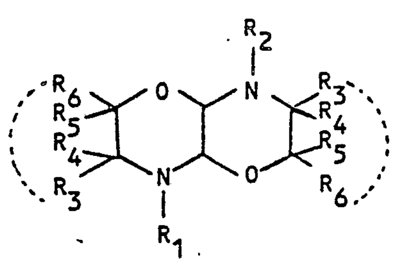

dans laquelle

$R_1$ et $R_2$, ayant des significations identiques ou différentes, représentent des groupes alkyle en $C_1$-$C_7$, cycloalkyle en $C_5$-$C_6$ ou phényle, étant spécifié que, lorsque $R_3$, $R_4$, $R_5$ et $R_6$ représentent tous l'hydrogène, les groupes n-butyle et phényle sont exclus,

$R_3$, $R_4$, $R_5$, $R_6$, ayant des significations identiques ou différentes, représentent l'hydrogène et/ou un substituant alkyle en $C_1$-$C_7$, un groupe phényle ou bien, par liaison de $R_3$ et $R_6$, un substituant cycloalkylène ou arylène (étant spécifié que, dans le cas d'un substituant arylène, les substituants $R_4$ et $R_5$ sont supprimés).

2. Composés selon la revendication 1, caractérisés en ce que

$R_1$, $R_2$ représentent un groupe $CH_3$, un groupe $C_2H_5$, un groupe cyclohexyle, un groupe phényle, étant spécifié que, lorsque $R_3$, $R_4$, $R_5$, $R_6$ représentent tous l'hydrogène, le groupe phényle est exclu,

$R_3$, $R_4$, $R_5$, $R_6$ représentent l'hydrogène ou bien

les groupes $R_3$ et $R_6$ forment, par liaison avec l'aminoéthanol, un noyau cyclohexane,

$R_4$, $R_5$ représentent l'hydrogène ou un groupe alkyle en $C_1$-$C_7$, de préférence l'hydrogène.

3. Composés selon les revendications 1 et 2, caractérisés en ce que

$R_1$ et $R_2$ = méthyle ou cyclohexyle et

$R_3$, $R_4$, $R_5$, $R_6$ = H, ou bien

$R_3$, $R_6$ = $-(CH_2)_4-$

$R_4$, $R_5$ = H.

4. Procédé de préparation des composés selon l'une des revendications 1 à 3, caractérisé en ce que l'on fait réagir le glyoxal avec des aminoalcools monosubstitués à l'azote de formule

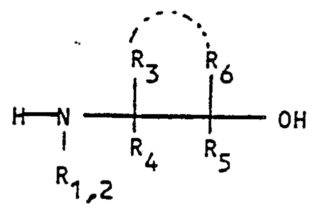

dans laquelle

$R_1$ à $R_6$ ont les significations indiquées dans les revendications 1, 2 ou 3, en solution aqueuse ou dans des solvants aqueux, de préférence à des températures de 40 à 100 °C et de préférence à un rapport molaire de 1 : 2.

5. Utilisation des composés selon l'invention, selon les revendications 1 à 3 en quantités de 0,1 à 10 % en poids dans des résines synthétiques contenant des groupes ester et/ou des groupes carbonate, en tant qu'agents de protection contre l'hydrolyse.

6. Utilisation selon la revendication 5, dans des polyuréthannes contenant des groupes ester et/ou des groupes carbonate, obtenus à partir de polyisocyanates, de polyols portant des groupes ester et/ou des groupes carbonate et le cas échéant d'autres produits auxiliaires et additifs, caractérisée en ce que l'on utilise en tant qu'agents de protection contre l'hydrolyse des 2,3,2',3'-bis-morpholines selon les revendications 1 à 3 en quantités de 0,1 à 10 % en poids par rapport au polyuréthanne.

7. Utilisation des bis-morpholines selon l'invention, selon les revendications 1 à 3 en tant qu'agents de protection contre l'hydrolyse avec utilisation conjointe d'agents de protection contre l'hydrolyse de type courant.